# EUROPEAN PATENT APPLICATION

(11) **EP 1 426 382 A1**
(43) Date of publication of application: **09.06.2004**
(21) Application number: 02027526.9
(22) Date of filing: 06.12.2002
(51) Int. Cl.: C07K 14/765, C07K 14/705, C07K 14/16, A61K 38/38, A61K 38/17, A61K 38/16, A61P 31/18

(54) **Antiviral human serum albumin**

(71) Applicant: Aventis Behring GmbH, 35002 Marburg (DE)
(72) Inventor: Friesen, Heinz-Jürgen, Dr., 35041 Marburg (DE)

(57) **Abstract**

An antiviral human serum albumin is described which HSA is prepared by fusion of human serum albumin with a protein or polypeptide being capable of inhibiting the fusion of virus-infected cells with non-infected cells, wherein the polarity of the amino groups of the fused HSA is chemically modified into a negative moiety.

## Description

The invention relates to antiviral human serum albumins and pharmaceutical preparations containing them which are suitable for treating or curing viral infections including orthomyxoviruses like influenza, paramyxoviruses like parainfluenza, measles and immunodeficiency diseases such as retrovirus infections, including AIDS and AIDS-related diseases, and which also can be used to inhibit the fusion of virus-infected cells with non-infected cells.

It is known from International patent application WO 92/15316 that pharmaceutical preparations are suitable for treating and curing of viral infections if they contain modified proteins or polypeptides as active substances which have acquired an additional net negative charge by derivatisation of their amino groups with a reagent which prevents protonation of their amino groups.

These results have been confirmed in US Patent 5.869.457 and it has been found for serum albumin that a correlation exists between the negative charge of the modified protein and its antiviral activity. The more negative the protein is charged by a suitable chemical modification, the greater is its antiviral activity, in particular its anti-HIV activity.

In addition several scientific publications have shown that a negatively charged albumin is a potent inhibitor of HIV-1 replication in vitro and that succinylated and acotinylated human serum albumins act at an early stage of the virus life cycle. It has been confirmed that by a direct interaction of negatively charged albumin with HIV-1 gp120 the binding of the virus through gp120 with CD4-cells is blocked.

It is now suggested to increase the antiviral effect of a negatively charged albumin by using an albumin which has been fused with a protein or polypeptide selected from the class of the "entry inhibitors".

Most preferred are such fusion proteins where in the albumin part 25% or more of the amino groups, carrying one positive charge each, have been converted to groups carrying one or two negative charges.

In cases where the presence of lysine in the entry inhibitor as part of such a fusion protein is deleterious to its desired activity, lysine may be replaced by a suitable other amino acid.

Entry inhibitors are agents that prevent viruses from infecting target cells, and within this class, at least three subclasses already exist. The penetration of cells by viruses like HIV is a multi-step process. At first HIV must recognize and bind to two separate molecules on the outer membrane of the target cell, CD4- and chemokine-receptors (CCR5 and/or CXCR4). Once the virus attaches itself to the cell surface, fusion occurs, the viral and cell membranes merge and the viral core is inserted into the cell cytoplasm. Each of these three events, CD4-receptor binding or attachment, chemokine- or co-receptor binding and fusion is an independent target for drug intervention.

The infection of a non-infected cell by an infected cell involves essentially the same steps.

Entry inhibitors to which a larger molecule like albumin has been attached exhibit a beneficial prolongated half-life time. This has been described e.g. in WO 01/79444.

Subject of the present invention is therefore a new antiviral human serum albumin which is prepared by fusion of human serum albumin (HSA) within a protein or polypeptide selected from the group of entry inhibitors which is capable of inhibiting the fusion of virus-infected cells with non-infected cells, wherein the polarity of the amino groups of the fused HAS is chemically modified into a negative moiety.

Examples of such entry inhibitors, which are polypeptides or proteins by nature, are CD4, T-20, T-1249, 5-Helix, DP-178 or cyanovirin. The scientific background of the similarities in the mechanism of the fusion steps of retroviruses like HIV and SIV and paramyxoviruses like measles are outlined in e.g. Lambert et al., PNAS 93, 2186-2191, 1996. Orthomyxovirus, i.e. influenza, replication is also inhibited by modified albumins. Entry inhibitors for HIV, SIV and measles are described e.g. in US Patents 6.020.459, 6.017.536, and 6.013.263, respectively. A compilation of entry inhibitors presently in development is disclosed in detail in the Datamonitor Report of February 2001 entitled "Entry Inhibitors in HIV" (www.datamonitor.com).

Albumin fusion products are described in International patent applications WO 9724445, 0179480, 0179442, 0179443, 0179444, 0179271 and 0179258 and US Patent 6.165.470 .Such an albumin fusion product may be obtained by the coupling, through chemical or genetic engineering techniques, of at least two distinct functions, namely a stable plasma transporter function provided by human serum albumin (HSA) and an entry inhibitor function, which prevents the invasion of the virus into the non-infected cell or the fusion of a virus infected cell with a non-infected cell. Such coupling processes are shown in US Patent 6.165.470 for the hybrid macromolecule comprising HSA-CD4 and for various other albumin fusions in International patent applications WO 9724445, 0179480, 0179442, 0179443, 0179444, 0179271 and 0179258, but can also be performed for the coupling of HSA with the entry inhibitors of polypeptide or protein nature like those mentioned above. All these hybrid macromolecules exhibit a biological activity against the fusion of virus or virus-infected cells with non-infected cells.

The antiviral potency of these compounds however can be considerably increased if such an albumin fusion protein containing a fusion inhibitor is substituted with negatively charged molecules by conversion with a dicarboxylic or tricarboxylic anhydride or attaching dicarboxylic or tricarboxylic acids via amide bonds or by other means like reacting corresponding activated esters or acid halides.

Known entry inhibitors act at different sites and thus exhibit variations of the basic mechanism of the fusion and entry inhibitor effects.

Cyanovirin-N preferably targets N-linked, high mannose glycans in an oligosaccharide-specific manner on e.g. gp120 in HIV or such structures in herpes viruses (Shenoy SR et al. 2001. Selective interactions of the HIV-inactivating protein, cyanovirin-N, targets high-mannose oligosaccharides on gp120 and other glycoproteins. J. Pharmacol. Exp. Ther. 2972-704-710; Bolmstedt AJ et al. 2001. Cyanovirin-N defines a new class of antiviral agent targeting N-linked, high mannose glycans in an oligosaccharide-specific manner. Mol. Pharmacol. 5905-949-954).

T-20 and T-1249 target the fusion process between viral and cellular membrane during virus entry into the cell. They are small molecules that bind to crucial helical structures in transition states involved in the fusion process thus providing an effective method of inhibiting the virus (for reviews see Chan DC and Kim PS. 1998. HIV Entry and its Inhibition, Cell 93:681-684; Doranz BJ 2000. The use of Envelope for HIV therapeutics: from Vaccines to Co-receptors. Emerging Therapeutic Targets 4:423-437; LaBranche CC 2001. HIV fusion and its inhibition. Antiviral Research 50:95-115) (Wild CT et al. 1994. Peptides corresponding to a predictive a-helical domain of human immunodeficiency virus type 1 gp41 are potent inhibitors of virus infection. Proc. Natl. Acad. Sci. 91:9770-9774).

The mechanism of the action of 5-Helix was described in 2001 (Root MJ 2001. Protein design of an HIV-1 entry inhibitor. Science 291:884-888). The binding of 5-Helix is thought to prevent the conformational changes on gp41 associated with the fusion event differently from T20 or T1249 and thereby also prevent infection of the cell.
The entry inhibitor and the modified albumin parts of these novel human serum albumin/entry inhibitor fusion products can interfere at different sites and times during the virus-cell-fusion step. Especially binding of these two parts at different sites with suitable proximity is beneficial as it improves on both, the binding kinetics and the binding strength. On the basis of this double-action mechanism, the entry inhibitor and the modified albumin parts show additive effects and improvements over these parts taken alone.

The fusion of entry inhibitor and albumin is a tool to provide enhanced half-life time compared especially to an entry inhibitor of lower molecular weight.

Preferred for such a chemical conversion are maleic anhydride or succinic anhydride as dicarboxylic anhydrides and cis- or trans-aconitic anhydride or citric anhydride as tricarboxylic anhydrides.

The modified human serum albumins which contain additional net negative charges can be used in the pharmaceutical preparations according to the invention. Compounds of this type are generally water-soluble and they can exist in ionic form and have a molecular weight of about 70,000. However, the molecular weight can also be appreciably higher or lower, the important factor is that a poly-anionic polypeptide structure is present in the molecule.

Medicinal preparations which contain the negatively charged antiviral HSA according to the invention can also be in the form of powders, suspensions, solutions, sprays, emulsions, ointments or creams and can be used for topical application, for intranasal, rectal or vaginal administration, and also for oral or parenteral (intravenous, intradermal, intramuscular, intrathecal, and the like) administration. Such preparations can be prepared by combining the negatively charged antiviral HSA polypeptide (for example by mixing, dissolving or the like) with pharmaceutically acceptable excipients of a neutral type, such as aqueous or non-aqueous solvents, stabilizers, emulsifiers, detergents and additives, and also, if desired, with colorants and aroma substances. The concentration of the negatively charged antiviral HSA in the preparation according to the invention can vary substantially and be between 0,001, preferably 0,1 and 100%, depending on the mode of administration. Furthermore, the dose of the negatively charged antiviral HSA to be administered can, for example, be between 0,1 mg and 100 mg/kg of body weight.

It is expected that by intravenous administration of the fused antiviral human serum albumin as described according to the present invention either by infusions of by repeated single injection usually with a total input per day of a least 1,5 mg/kg body weight and maximally 15 mg/kg, long lasting antiviral concentrations will be attained in the blood stream and the lymphatic system of virus infected patients.

The invention also relates to the use of the antiviral HSA according to the invention which has acquired additional net negative charges, as defined above, as active substance or as substance contributing to the action, or as carrier for active substance when preparing pharmaceutical preparations and immunodeficiency diseases such as retrovirus infections, including AIDS and AIDS-related diseases, and which can be used to inhibit fusion of infected with non-infected cells.

The invention also relates to a method for treating diseases or disorders caused by retrovirus infections such as AIDS and AIDS-related diseases, or conditions in which fusion of virus with cells or fusion of virus-infected cells with non-infected cells occurs, with which method an additionally negatively charged HSA as defined above is used.

The invention also relates to a method for the preparation of antiviral HSA with a reagent which prevents protonation of basic amino acids, the proteins or polypeptides acquiring additional net negative charges such that the prolongation of the retention time of the antiviral HSA according to the invention compared with that of an antiviral HSA, which is not negatively charged is at least four minutes, the retention time being determined by means of a FPLC system (Amersham-Pharmacia) on an anion exchange column, as described by Jansen et al., Mol. Pharmacology 39: 818-823, 1991 and Mol. Pharmacology 44: 1003-1007, 1993.

According to the invention, it is suggested that the negatively charged antiviral HSA is, inter alia, a powerful and selective anti-HIV agent which substantially reduces syncytium formation and virus-cell fusion, does not bind to the OKT4A epitope of the CD4 receptor and has only a slight inhibitory action on the interaction of anti-gp120 and mAb gp120 and the binding of virus cells at very low concentrations. The negatively charged antiviral HSA used or prepared according to the invention has no or only a low toxicity.

The invention is illustrated with reference to the following examples

### a) Preparation of negatively charged, antiviral HSA according to the invention

A T20-fusion macromolecule prepared in accordance with U.S. patent 6.165.470 was modified by treatment with succinic acid anhydride which reacts with the amino groups of the T20-HSA molecule. As a result the T20-HSA molecules acquires additional net negative charges. This derivatisation has the effect that the retention time of the T20-HSA molecule on an anion exchange column is prolonged for at least four minutes compared with the retention time of the non-succinylated fusion molecule, the retention time being determined by means of a FPLC system (=Fast Protein Liquid Chromatography) from Amersham-Pharmacia on an anion exchange column as described by Jansen et al., Mol. Pharmacology 44: 1003-1007, 1993.

The succinylation was performed according to Jansen et al., Mol. Pharmacology 39: 818-823, 1991 as follows:

### Modified T20-HSA

100 mg of T20-HSA were dissolved in 10 ml of 0,2 M K₂HPO₄ (pH 8.0). 100 mg of solid succinic anhydride were added and the solution was stirred until the succinic anhydride had dissolved. The pH was kept between 8.0 und 8.5 using 6 M or 4 M NaOH.

In order to remove insoluble material the solution was filtered through a filter with 0,2 µm openings, purified on a Sephadex G25 column, washed with distilled water on a PM 10 membrane in an Amicon "Stirred Cell Concentrator" and finally lyophilized.

### b) Anti-HIV-1-Test in the presence of the antiviral HSA according to the invention

Jurkat cells (e.g. 5x10⁵/ml) were mixed with HIV inhibitor or control protein solution and HIV-1 (e.g. 1.5x10⁵ CCID₅₀/ml), each sample being assayed in 8 replicates in 98-well plates, and incubated at 37°C for 4 days. Each sample was evaluated for the amount of cell-cell fusions, antiviral activity being measured through reduction of fusion events compared to control.

## Claims

1. Anti-viral human serum albumin which is prepared by fusion of human serum albumin (HSA) with a protein or polypeptide being capable of inhibiting the fusion of virus-infected cells with non-infected cells, wherein the polarity of the amino groups of the fused HSA is chemically modified into a negative moiety.

2. Antiviral human serum albumin as claimed in claim 1, wherein the polarity of the amino groups of the fused HSA is chemically modified by converting at least 25% of the amino groups of the albumin part with a dicarboxylic or tricarboxylic anhydride, activated ester or acid halide of a dicarboxilic or tricarboxilic acid.

3. Antiviral human serum albumin as c laimed in claims 1 and 2, wherein the dicarboxylic anhydride is maleic anhydride or succinic anhydride, and the tricarboxylic anhydride is cis- or trans-aconitic anhydride or citric anhydride.

4. Antiviral human serum albumin as claimed in claims 1 to 3, wherein the protein capable of inhibiting the fusion of virus-infected cells with non-infected cells is selected from the group of virus fusion inhibitors or entry inhibitors, which prevent viruses from infecting targeted cells, comprising, CD4-albumin, T20-albumin, 5-helix-albumin, DP-178-albumin, cyanovirin-albumin or T-1249-albumin.

5. Pharmaceutical preparation for treating or curing viral infections comprising the antiviral human serum albumin of claims 1 to 4 as an active substance or as a substance contributing to the action or as a carrier for other substances, which are capable of inhibiting the fusion of virus-infected cells with non-infected cells.

6. Method of treating viral diseases or disorders caused by a virus or retrovirus infection, wherein a pharmaceutical preparation as claimed in claim 5 is administered to a patient suffering from such a viral infection.

7. Process for the preparation of an antiviral human serum albumin as claimed in claims 1 to 4, wherein the at least 25% of the amino groups of the albumin part of the fused HSA are derivatised with a reagent which prevents the protonation of the amino groups, the fused HSA acquiring additional net negative charges such that the prolongation of the retention time of the fused HSA derivative compared with that of the fused HSA not converted to a derivative is at least four minutes, the retention time being determined by means of Fast Protein Liquid Chromatography (FPLC).
